(19) Europäisches Patentamt

European Patent Office

Office européen des brevets

(11) **EP 1 019 544 B1**

(12) **EUROPÄISCHE PATENTSCHRIFT**

(45) Veröffentlichungstag und Bekanntmachung des Hinweises auf die Patenterteilung:
**28.08.2002 Patentblatt 2002/35**

(51) Int Cl.⁷: **C12Q 1/68**

(21) Anmeldenummer: **98954303.8**

(22) Anmeldetag: **30.09.1998**

(86) Internationale Anmeldenummer:
**PCT/EP98/06219**

(87) Internationale Veröffentlichungsnummer:
**WO 99/016901 (08.04.1999 Gazette 1999/14)**

(54) **VERFAHREN ZUM VERGLEICH DER KOPIENZAHL VON NUKLEINSÄURESEQUENZEN**

METHOD FOR COMPARING THE NUMBER OF REPLICATIONS OF NUCLEIC ACID SEQUENCES

PROCEDE POUR COMPARER LE NOMBRE DE REPLIQUES DE SEQUENCES D'ACIDE NUCLEIQUE

(84) Benannte Vertragsstaaten:
**AT BE CH DE DK ES FI FR GB GR IE IT LI LU NL PT SE**

(30) Priorität: **30.09.1997 DE 19743301**

(43) Veröffentlichungstag der Anmeldung:
**19.07.2000 Patentblatt 2000/29**

(73) Patentinhaber: **MetaSystems Hard & Software GmbH**
**68804 Altlussheim (DE)**

(72) Erfinder:
• **LÖRCH, Thomas**
**D-68799 Reilingen (DE)**
• **PLESCH, Andreas**
**D-68723 Schwetzingen (DE)**
• **ISOLA, Jorma Laboratory of Cancer Genetics**
**2 kerros 33520 Tampere (FI)**

(74) Vertreter: **Müller-Boré & Partner Patentanwälte**
**Grafinger Strasse 2**
**81671 München (DE)**

(56) Entgegenhaltungen:
**WO-A-93/18186**        **WO-A-96/17958**

• **KALLIONIEMI O -P ET AL: "OPTIMIZING COMPARATIVE GENOMIC HYBRIDIZATION FOR ANALYSIS OF DNA SEQUENCE COPY NUMBER CHANGES IN SOLID TUMORS" GENES, CHROMOSOMES & CANCER, Bd. 10, Nr. 4, August 1994, Seiten 231-243, XP000606237**
• **VISAKORPI T. ET AL.,: "Genetic changes in primary and recurrent prostate cancer by comparative genomic hybridization" CANCER RESEARCH, Bd. 55, 15. Januar 1995, Seiten 342-347, XP002092365**

**Beschreibung**

Die Erfindung bezieht sich auf ein molekulargenetisches Verfahren. Mit diesem Verfahren wird die Kopienzahl verschiedener Nukleinsäuresequenzen eines zu untersuchenden Kollektivs von Nukleinsäuresequenzen, eines sogenannten Nukleinsäuresequenzkollektivs, mit der Kopienzahl eines anderen Nukleinsäuresequenzkollektivs verglichen. Durch diesen Vergleich werden die Veränderungen in der Kopienzahl der Nukleinsäuresequenzkollektive relativ zueinander sowie die Stellen, an denen diese Veränderungen auftreten, bestimmt.

Die hier beschriebenen Nukleinsäuresequenzkollektive können insbesondere Genome oder Chromosomen bzw. DNA- oder RNA-Sequenzen aus verschiedenen Genomen sein. Die weiteren Ausführungen, soweit sie sich auf Genome, Chromosomen oder DNA- oder RNA-Sequenzen beziehen, sollen in keinerlei Hinsicht eine Einschränkung des allgemeinen Begriffs des Nukleinsäuresequenzkollektivs darstellen, sondern werden ausschließlich zur vereinfachten Darstellung verwendet. Selbstverständlich gilt das im Rahmen der vorliegenden Erfindung Offenbarte generell für beliebige Nukleinsäuresequenzkollektive.

Die Fluoreszenzmarkierung von Nukleinsäuren bzw. Chromosomen und der anschließende fluoreszenzmikroskopische Nachweis sind bereits seit 1970 aus Veröffentlichungen von Person und Bobrow bekannt

Auch die Herstellung von In-situ-Hybridisierungen unter Verwendung von Fluoreszenzmarkern wird zum Beispiel in der US 5 447 841, der EP 430 402 A1 und der WO 90/05789 beschrieben. Diese Fluoreszenz-in-situ-Hybridisierung wird vor allem zur Analyse numerischer und struktureller chromosomaler Anomalien angewandt. Hierbei werden fluoreszenzmarkierte DNA- Sequenzen auf eine zu untersuchende DNA hybridisiert. Die markierte DNA lagert sich durch Rekombination an der Stelle der zu untersuchenden DNA an, die komplementär zur markierten DNA ist. Die hybridisierten markierten DNA-Sequenzen lassen sich dann mit fluoreszenzmikroskopischen Techniken nachweisen. Dieser Nachweis der Fluoreszenzmarker wird in Abhängigkeit von der Fragestellung an Metaphasechromosomen oder Interphasekernen durchgeführt. Für den Fall, daß die Lokalisierung der markierten DNA-Sequenzen auf dem entsprechenden Chromosom von Bedeutung ist, werden die Metaphase-Zellen untersucht. Soll aber die Anzahl der markierten DNA-Sequenzen festgestellt werden, wie beispielsweise bei der Analyse numerischer Aberrationen der Chromosomen, so genügt es, die Interphasekerne der fluoreszenzmikroskopischen Analyse zu unterziehen.

Eine Weiterentwicklung dieser Technik ist die vor allem in der Tumordiagnostik verwendete vergleichende Genomhybridisierung (comparative genomic hybridization, CGH). Bei diesem Verfahren wird im Gegensatz zu dem oben genannten Verfahren die isolierte DNA eines zu untersuchenden Genoms mit Fluoreszenzfarbstoffen markiert. Diese markierte DNA wird zusammen mit Referenz-DNA auf Metaphasechromosomen hybridisiert.

So wird beispielsweise aus Tumorgewebe isolierte, zu untersuchende DNA (Test-DNA) in einer Fluoreszenzfarbe (z.B. grün) markiert. Diese markierte Test-DNA wird dann zusammen mit normaler Referenz-DNA, die in einer anderen Farbe markiert ist (z.B. rot), auf normale Metaphasechromosomen hybridisiert, die beispielsweise mit 4,6-Diamidino-2-phenylindol (DAPI) blau markiert werden. Bei der Rekombination während der Hybridisierung findet eine Konkurrenz der Test-DNA und der Referenz-DNA um die Bindungsstellen auf der DNA der Metaphasen-Zelle statt. Normale Bereiche des Tumor-Genoms werden sich mit der gleichen Wahrscheinlichkeit an die Metaphase-Chromosomen anlagern wie die entsprechenden Bereiche der Referenz-DNA. Diese Bereiche weisen bei der fluoreszenzmikroskopischen Auswertung ein bestimmtes Intensitätsverhältnis der verwendeten Farbstoffe rot und grün auf. Die Bereiche des Tumor-Genoms, die eine Vermehrung der Kopienzahl der DNA-Sequenzen aufweisen, rekombinieren mit höherer Wahrscheinlichkeit als die Referenz-DNA mit den ihnen komplementären Stellen der Metaphase-Chromosomen, so daß es an den entsprechenden Stellen zu einer Verschiebung des Intensitätsverhältnisses nach grün kommt. Dagegen werden die Bereiche der Test-DNA, in denen die Kopienzahl der DNA-Sequenzen vermindert ist, anhand einer Rotverschiebung des Intensitätsverhältnisses erkannt. Die unterschiedlichen Fluoreszenzintensitäten, anhand derer die Veränderungen in der Kopienzahl der DNA-Sequenzen erkennbar sind, werden über eine quantitative Auswertung der Grün/Rot-Intensitätsverhältnisse als sogenannte Profile der Ratio-Werte dargestellt. Dabei stellt ein Ratio-Wert den normierten Quotienten aus den Intensitäten der grünen und der roten Fluoreszenz einer bestimmten DNA-Sequenz dar.

In der WO 93/18186 wird ein Verfahren der vergleichenden Genom-Hybridisierung beschrieben. Bei diesem Verfahren wird die DNA eines zu untersuchenden Genoms mit Fluoreszenzfarbstoffen markiert und auf Metaphasechromosomen hybridisiert. Anschließend wird die Intensität der fluoreszenzmikroskopischen Signale der zu untersuchenden DNA in Abhängigkeit von der Position auf den Metaphasechromosomen gemessen. Durch den Vergleich der Intensitäten an verschiedenen Stellen des zu untersuchenden Genoms, die verschiedenen DNA-Sequenzen entsprechen, können Rückschlüsse auf die Existenz solcher DNA-Sequenzen gezogen werden, die relativ zu den übrigen DNA-Sequenzen des Genoms mit einer veränderten Anzahl von Kopien vorhanden sind.

Bei diesem CGH-Verfahren ergeben sich nun in der Praxis systematisch Abweichungen von den erwarteten Ratio-Werten. So findet sich beispielsweise für das X-Chromosom regelmäßig folgender Sachverhalt:

Das X- Chromosom wird als interne Kontrolle genutzt, wenn weibliche Tumor-DNA (zwei X-Chromosomen) mit männlicher Referenz-DNA (nur ein X-Chromosom) gemischt und auf Metaphasechromosomen hybridisiert wird. Der erwartete Ratio-Wert müßte sich aufgrund der doppelten Anzahl der X-Chromosomen in der Test-DNA im Mittel bei 2,0 befinden. Er liegt aber bei den meisten Hybridisierungen deutlich darunter, etwa nur zwischen 1,5 und 1, 8. Allgemein wird eine Verringerung der Dynamik der CGH Ratio-Werte beobachtet, d.h. sowohl die Ratio-Werte <1 als auch die Ratio-Werte >1 sind in Richtung 1, also in Richtung Normalwert, verschoben. Dieser Dynamikverlust führt zu einer reduzierten Empfindlichkeit des CGH-Verfahrens, geringere Veränderungen der Kopienzahl können nicht mehr zuverlässig erfaßt werden, da sie unterhalb der Signifikanzschwellen bleiben. Die diagnostische Interpretierbarkeit der CGH-Ergebnisse ist dadurch stark eingeschränkt, insbesondere wenn es sich um geringe Veränderungen in der Anzahl der Kopien handelt.

Die genaue Ursache der verringerten Dynamik der Ratio-Profile ist nicht bekannt. Eine mögliche Erklärung für den beobachteten Effekt wäre eine unzureichende Absättigung der nicht-spezifischen Anteile der eingesetzten Test- und Referenz-Genome. Nicht-spezifische Anteile, sogenannte repetitive Sequenzen, sind Stellen des Genoms, die häufig vorkommende DNA-Sequenzen aufweisen und deshalb mit verschiedenen anderen Sequenzen innerhalb des Genoms rekombinieren können. Die additive Komponente dieser unspezifischen Rekombination für Test- und Referenz-DNA führt zu einer Verschiebung der Ratio-Werte in Richtung 1. Bei der Durchführung von CGH-Untersuchungen werden diese repetitiven Sequenzen zwar durch nicht-markierte Cot-1-DNA abgesättigt, diese Absättigung erfolgt jedoch nicht immer vollständig.

Die Korrektur dieses Effekts ist bisher von Lundsteen et al, derart vorgeschlagen worden, daß ein empirisch ermittelter Wert, wie beispielsweise 10 %, von den gemessenen Fluoreszenzintensitäten subtrahiert wird. Ein entscheidender Nachteil dieser empirischen Korrektur ist jedoch die Anfälligkeit gegen experimentelle Schwankungen. Auch haben Ergebnisse gezeigt, daß ein solch einfaches Modell die beobachteten Effekte nicht ausreichend beschreibt. Karhu et al. in "Quality Control of CGH: Impact of Metaphase Chromosomes and the Dynamic Range of Hybridization", Cytometry, 28: S. 198 - 205, (1997) zeigen, daß der Dynamikbereich der Ratio-Werte für identische Test- und Referenz-DNA stark von der Hybridisierbarkeit der Metaphasechromosomen, auf die hybridisiert wird, abhängt und nicht mit üblichen Überprüfungskriterien der Unterdrückung repetitiver Sequenzen, wie Hybridisierungsintensität der Zentromere, korreliert.

In der WO 93/18186 wird in Anspruch 10, 11 und 17 auch beschrieben, die veränderte Anzahl der Kopien von DNA-Sequenzen eines Genoms im Vergleich zu denjenigen eines anderen Genoms quantitativ zu bestimmen. Hierzu wird die DNA beider Genome in unterschiedlichen Fluoreszenzfarben markiert und auf normale Metaphasechromosomen hybridisiert. Nach der Messung der Intensitäten in Anbhängigkeit von ihrer Position auf den Metaphasechromosomen wird ein Profil der Ratio-Werte gebildet, das die relativen Intensitäten der DNA-Sequenzen der eingesetzten Genome zueinander an jeweils entsprechenden Stellen der Genome wiedergibt. Das erhaltene Ratio-Profil wird anhand einer auf beiden Genomen bekannten Kalibriersequenz normiert, um danach die unbekannten Sequenzen über ihre Ratio-Werte in absolute Kopienzahlen umrechnen zu können. Eine Korrektur der experimentell beobachteten Verringerung der Dynamik von CGH Ratio-Profilen ist auf diese Weise nicht möglich. Auch die dort in Anspruch 12 beschriebene Kalibrierung von Kopienzahlveränderungen über den paarweisen Vergleich von mehr als zwei gleichzeitig hybridisierten Genomen dient lediglich zur Umrechnung von Quotienten in Absolutwerte.

Es ist die Aufgabe der vorliegenden Erfindung, ein Verfahren zum Vergleich der Kopienzahl von Nukleinsäuresequenzen von verschiedenen Nukleinsäuresequenzkollektiven anzugeben, das diese Dynamikverringerung nicht aufweist. Es soll also ermöglicht werden, durch Verwendung eines internen Standards systematische Hybridisierungseinflüsse zu bestimmen und zu korrigieren. Weiterhin soll die Korrektur unabhängig von den zu untersuchenden Nukleinsäuresequenzkollektiven durchgeführt werden können, so daß ein Nukleinsäuresequenzkollektiv untersucht werden kann, das komplett unbekannt ist.

Eine Lösung der beschriebenen Aufgabe wird durch das in Anspruch 1 bezeichnete Verfahren angegeben.

Dazu werden zum Vergleich der Kopienzahl von Nukleinsäuresequenzen zweier oder mehrerer Nukleinsäuresequenzkollektive folgende Schritte durchgeführt:

a) Hybridisierung von

i) einem zu untersuchenden Nukleinsäuresequenzkollektiv (T),
ii) einem Referenz-Nukleinsäuresequenzkollektiv (R) und
iii) mindestens einem weiteren Nukleinsäuresequenzkollektiv mit bekannter Abweichung in der Kopienzahl der Nukleinsäuresequenzen an mindestens einer bekannten Stelle (Eich-Nukleinsäuresequenzkollektiv, S), wobei das weitere Nukleinsäuresequenzkollektiv dem Referenz-Nukleinsäuresequenzkollektiv möglichst ähnlich ist,

wobei die Nukleinsäuresequenzkollektive mit unterscheidbaren Markierungen versehen sind, auf ein Kollektiv geeigneter Ziel-Nukleinsäuresequenzen,
b) Nachweis der Intensitäten der Markierungen der einzelnen, auf die Ziel-Nukleinsäuresequenzen hybridisierten markierten Nukleinsäuresequenzkollektive,

c) Bestimmung der Quotienten $Q_R$ der Intensitäten der Markierungen der Nukleinsäuresequenzen des zu untersuchenden Nukleinsäuresequenzkollektivs und den entsprechenden Intensitäten der Markierungen der Nukleinsäuresequenzen des Referenz-Nukleinsäuresequenzkollektivs,

d) Bestimmung der Quotienten $Q_S$ der Intensitäten der Markierungen der Nukleinsäuresequenzen des zu untersuchenden Nukleinsäuresequenzkollektivs und den entsprechenden Intensitäten der Markierungen der Nukleinsäuresequenzen des Eich-Nukleinsäuresequenzkollektivs,

e) Bestimmung der relativen Kopienzahlen, der sogenannten Ratio-Werte, von den zu untersuchenden Nukleinsäuresequenzen gegenüber den Referenz-Nukleinsäuresequenzen durch geeignete Normierung der Quotienten $Q_R$ aus c),

f) Bestimmung der relativen Kopienzahlen, der sogenannten Ratio-Werte, von den zu untersuchenden Nukleinsäuresequenzen gegenüber den Eich-Nukleinsäuresequenzen durch geeignete Normierung der Quotienten $Q_S$ aus d),

g) Bestimmung von wenigstens zwei Stützstellen, die wenigstens zwei im Betrag voneinander abweichenden Ratio-Werten der Eich- und der Referenz-Nukleinsäuresequenzen mit bekannter Abweichung in der Kopienzahl der Eich-Nukleinsäuresequenzen entsprechen,

h) Berechnung einer Korrekturkurve anhand der an den Stützstellen vorhandenen Abweichungen der erhaltenen Ratio-Werte von den aufgrund der bekannten Veränderungen in der Kopienzahl der Eich-Nukleinsäuresequenzen erwarteten Ratio-Werten, und

i) Korrektur der unter e) erhaltenen Kopienzahlen bzw. Ratio-Werte mit Hilfe der unter h) erhaltenen Korrekturkurve.

Gemäß Anspruch 2 werden zur Markierung insbesondere unterschiedliche Fluoreszenzfarbstoffe verwendet. Die markierten Nukleinsäuresequenzen können anschließend durch die Messung ihrer fluoreszenzmikroskopischen Signale einfach nachgewiesen bzw. ausgewertet werden.

Es sind aber auch alle anderen Arten der Markierung denkbar.

Als Kollektiv der Ziel-Nukleinsäuresequenzen eignen sich vor allem Metaphase-Chromosomen oder isolierte Nukleinsäuresequenzen, die nach Anspruch 3 auf einem Substrat in definierter Weise angeordnet sind.

Diese Ziel-Nukleinsäuresequenzen können entsprechend Anspruch 5 bzw. 6 DNA oder cDNA sein.

Die in Anspruch 1 unter f) genannte geeignete Normierung der Quotienten $Q_R$ und $Q_S$ erfolgt beispielsweise durch Bildung der Quotienten aus den Werten $Q_R$ bzw. $Q_S$ und den insgesamt am häufigsten vorkommenden Werten für $Q_R$ bzw. $Q_S$, so daß der häufigste normierte Quotient den numerischen Wert 1,0 erhält.

Diese Normierung kann auch in folgenden 3 Schritten durchgeführt werden:

1) die Intensitäts-Profile der Nukleinsäuresequenzen des zu untersuchenden Nukleinsäuresequenzkollektivs werden auf einen Mittelwert von 1.0 normiert,

2) die Intensitäts-Profile der Nukleinsäuresequenzen des Referenz-Nukleinsäuresequenzkollektivs werden so normiert, daß der häufigste Ratio-Wert aus zu untersuchenden und Referenz-Nukleinsäuresequenzen den Wert 1.0 zugewiesen bekommt,

3) die Intensitätsprofile der Nukleinsäuresequenzen des Eich-Nukleinsäurekollektivs werden unabhängig in entsprechender Weise wie die Nukleinsäuresequenzen des Referenz-Nukleinsäuresequenzkollektivs normiert.

Gemäß Anspruch 7 können beispielsweise als Referenz-Nukleinsäuresequenzkollektiv normale weibliche DNA, als Eich-Nukleinsäuresequenzkollektiv eine männliche Zellinie mit einer Trisomie 13 verwendet werden. Die theoretischen mittleren Ratio-Werte aus Eich- und Referenz-Nukleinsäuresequenzkollektiv für die Profile zweier Chromosomen sind daher bekannt (X-Chromosom: 0.5, Chromosom 13: 1.5). Die tatsächlichen Ratio-Werte differieren aufgrund der Dynamikabweichung. Aus den bekannten Ratio-Werten der Chromosomen 13 und X, sowie aus dem mittleren Ratio-Wert für alle übrigen, im Eich-Nukleinsäuresequenzkollektiv in normaler Kopienzahl vorliegenden Chromosomen, der aufgrund der Normierung immer 1.0 ist, können die Korrekturterme berechnet und auf die aus den gemessenen Intensitäts-Profilen erhaltenen Ratio-Werte angewendet werden, um dadurch die Reproduzierbarkeit und Empfindlichkeit der CGH-Methode zu verbessern.

Hierbei werden den verwendeten Zeichen folgende Bedeutungen zugeordnet:

| | |
|---|---|
| $t_i$ | der gemessene Wert des Test-DNA-Intensitätsprofils an der Stelle i |
| $r_i$ | der gemessene Wert des Referenz-DNA-Intensitätsprofils an der Stelle i |
| $s_i$ | der gemessene Wert des Eich-DNA-Intensitätsprofils an der Stelle i |
| $t'_i$ | der korrigierte Wert des Test-DNA-Intensitätsprofils an der Stelle i |
| $r'_i$ | der korrigierte Wert des Referenz-DNA-Intensitätsprofils an der Stelle i |
| $s'_i$ | der korrigierte Wert des Eich-DNA-Intensitätsprofils an der Stelle i |
| $q_i$ | der Wert des Ratioprofils an der Stelle i |

$q'_i$          der korrigierte Wert des Ratioprofils an der Stelle i

$A, B, C, M, A_X, A_{13}, M_X, M_{13}$     die Korrekturterme, die nach den nachstehenden Algorithmen berechnet werden

**I** Additive Korrektur aus X-Chromosom:

Diese Korrektur geht von einer Erhöhung aller gemessenen Hybridisierungsintensitäten (etwa aufgrund von nicht vollständig unterdrückten repetitiven Sequenzen) aus, was alle Ratio-Werte in Richtung 1.0 verschiebt und dadurch die Dynamik der Ratio-Profile reduziert. Dies kann durch eine additive Korrektur der einzelnen Intensitätsprofile vor der Ratio-Berechnung korrigiert werden. Die Korrekturformel lautet:

$$t'_i = t_i + A$$

$$r'_i = r_i + A$$

Die Ratio-Werte werden mit Hilfe der korrigierten Profil-Werte berechnet:

$$q'_i = t'_i/r'_i$$

Da der korrigierte Ratio-Wert für das X-Chromosom gleich dem theoretischen Wert 0.5 sein muß, folgt für den Korrekturterm A:

$$0.5 = s'_X / r'_X = (s_X + A) / (r_X + A)$$

$$A = r_X - 2 s_X$$

**II** Multiplikative Korrektur aus X-Chromosom:

Bei dieser Korrekturmethode wird davon ausgegangen, daß der Dynamikbereich der Ratio-Profile durch einen multiplikativen Faktor M reduziert wird, der die Ratio-Werte näher zum Wert 1.0 verschiebt. Der Wert 1.0 ist wiederum aufgrund der Normierung immer korrekt. Die Korrekturformel für den multiplikativen Faktor M ergibt sich zu:

$$(q'_i - 1) = M (q_i - 1)$$

oder

$$q'_i = M(q_i - 1) + 1$$

aus dem bekannten Ratio-Wert für Eich- und Referenz-Nukleinsäuresequenzen des X-Chromosoms folgt:

$$0.5 = M (s_X / r_X - 1) + 1$$

$$M = r_X / 2 (r_X - s_X)$$

**III** Quadratische Korrektur aus Chromosomen X, 13:

Bei diesem Korrekturansatz wird eine linear-quadratische Beziehung zwischen ermittelten und theoretischen Ratio-Werten angenommen. Die Korrekturformel lautet:

$$q'_i = A\, q_i^2 + B\, q_i + C$$

Aufgrund der Normierung gilt (der korrigierte Ratio-Wert für 1 ist 1):

$$1 = A + B + C$$

Die bekannten Ratio-Werte für Eich- und Referenz-Nukleinsäuresequenzen der Chromosomen X und 13 definieren zwei zusätzliche Beziehungen:

$$0.5 = A\, (s_X/r_X)^2 + B\, (s_X/r_X) + C$$

$$1.5 = A\, (s_{13}/r_{13})^2 + B\, (s_{13}/r_{13}) + C$$

Lösung dieses linearen Systems von 3 Gleichungen ergibt die Koeffizienten A, B und C. Wird ein Eich-Nukleinsäuresequenzkollektiv eingesetzt, das außer den Ratio-Werten 0.5, 1.0 und 1.5 noch weitere unterschiedliche Kopienzahlen bzw. Ratio-Werte aufweist, so ergeben sich, wie in Anspruch 9 beschrieben, zusätzliche Stützstellen für die Berechnung der Korrekturkurve. Aus den resultierenden Gleichungssystemen von 4 oder mehr verschiedenen Ratio-Stützstellen können dann Korrekturkurven dritter oder höherer Ordnung berechnet werden, die eine noch genauere Dynamikkorrektur ermöglichen.

**IV** Minimal-additive Korrektur aus Chromosomen X, 13:

Dieser Algorithmus berechnet einen additiven Korrekturfaktor aus dem X-Chromosom und einen zweiten, unabhängigen Korrekturfaktor aus dem Chromosom 13 und verwendet den kleineren Korrekturfaktor als konservative Schätzung, um Überkorrigieren zu vermeiden. Die Korrekturformel ergibt sich zu (vgl auch I):

$$t'_i = t_i + A$$

$$r'_i = r_i + A$$

Der Korrekturterm $A_X$ aus dem Ratio-Wert des X-Chromosoms ist (vgl. I):

$$0.5 = s'_X / r'_X = (s_X + A_x) / (r_X + A_x)$$

$$A_x = r_X - 2\, s_X$$

Für den Korrekturterm $A_{13}$ abgeleitet von Chromosom 13 gilt:

$$1.5 = s'_{13}/r'_{13} = (s_{13} + A_{13}) / (r_{13} + A_{13})$$

$$A_{13} = 2\, s_{13} - 3\, r_{13}$$

Die Korrektur verwendet den betragsmäßig kleineren der beiden Werte $A_X$ und $A_{13}$, um eine Überkorrektur zu vermeiden.

**V** Minimal Multiplikative Korrektur aus Chromosomen X, 13:

Analog zum Vorgehen unter II werden aus den Chromosomen X und 13 zwei unabhängige Korrekturfaktoren

$M_X$ und $M_{13}$ ermittelt, von denen der betragsmäßig kleinere für die Korrektur herangezogen wird, um eine Überkorrektur zu vermeiden.

Zusätzlich zur Dynamikkorrektur der CGH-Profile liefern die Normal-Ratio-Werte für Referenz- und Eich-Nukleinsäuresequenzen Informationen über die Reproduzierbarkeit und Konstanz der Hybridisierung bzw. über die differentielle Hybridisierbarkeit auf einzelne Bereiche der Ziel-Nukleinsäuresequenzen . Diese Information dient dazu, Bereiche, die mit erhöhter Wahrscheinlichkeit Hybridisierungsprobleme aufweisen, wie z.B. 1pter, 19 und 22 der Chromosomen, zu erkennen und bei der Interpretation der Ergebnisse entsprechend zu berücksichtigen.

Obwohl die Algorithmen beispielhaft für eine Korrektur anhand der ganzen Chromosomen X und 13 dargestellt wurden, ist es offensichtlich, daß die Korrekturkurven in analoger Weise anhand der Ratio-Profile von in ihrer Kopienzahl veränderten Chromosomenbereichen anstelle ganzer Chromosomen ermittelt werden können. In diesem Fall würden anstelle der Ratio-Profile der ganzen Chromsomen X bzw. 13, die entsprechenden Ratio-Profile von bekannt aberrierten Chromsomenbereichen für die Berechnung zugrundegelegt.

Hierzu sei folgendes Beispiel genannt: Wird ein Eich-Nukleinsäuresequenzkollektiv verwendet, das bekannte Kopienzahländerungen des p-Arms von Chromosom 1 und des q-Arms von Chromosom 12 aufweist, so wären die Indizes X und 13 durch 1p und 12q, und ggf. 0.5 und 1.5 durch die aktuellen Kopienzahländerungen der Chromosomenbereiche 1p und 12q des Eich-Nukleinsäuresequenzkollektivs zu ersetzen. Der grundsätzliche Korrekturansatz ist dadurch nicht betroffen.

Die Erfindung kann vorteilhaft auch auf CGH Untersuchungen an DNA-Arrays anstelle von Metaphasechromosomen als Hybridisierungs-Target angewandt werden. Solche DNA-Arrays sind in DE 43 44 726 A1 beschrieben und ersetzen Metaphasechromosomen durch eine definierte Anordnung von isolierten Nukleinsäuresequenzen auf einem geeigneten Substrat, die bestimmten Chromosomen oder Chromosomenabschnitten bis hinunter zu einzelnen Genen entsprechen können. Diese definierten Anordnungen werden z.B. auch in Anspruch 4 erwähnt. Die genannten Arrays lassen sich insbesondere vorteilhaft einsetzen, um die Ortsauflösung des CGH-Verfahrens zu erhöhen und um die Automatisierung der Auswertung zu erleichtern. Durch die getrennte, über eine relativ große Fläche verteilte Anordnung der Nukleinsäuresequenzen ist die Hybridisierbarkeit der DNA-Arrays im Vergleich zu Metaphasechromosomen sehr stark heruntergesetzt. Auch ist die Beurteilung der Hybridisierungsqualität sehr viel schwieriger als im Metaphasen- bzw. Chromosomen-Kontext. Eine interne Kontrolle und ggf. Korrektur der Hybridisierbarkeit der einzelnen Targets ist bei diesem Verfahren daher von besonderer Bedeutung, insbesondere, wenn geringe Veränderungen der Kopienzahl bestimmt werden sollen.

Eine weitere Ausbildung des erfindungsgemäßen Verfahrens nach den Ansprüchen 1 bis 8 ist in Anspruch 9 beschrieben. Hier werden in Schritt g) als Stützstellen mehr als zwei im Betrag voneinander abweichende Ratio-Werte verwendet, um die Berechnung von Korrekturkurven höherer Ordnung zu ermöglichen. Diese Ausgestaltung des erfindungsgemäßen Verfahrens ist bereits bei der Beschreibung der verwendeten Algorithmen I bis V erwähnt.

Im Grenzfall könnte als Eich-Nukleinsäuresequenzkollektiv ein Eich-Genom eingesetzt werden, das für mehrere Chromosomenbereiche bekannte numerische Aberrationen aufweist. Dabei ist jedoch der Gesamtanteil der aberrierten Chromosomenbereiche in dem Eich-Genom so zu beschränken, daß der am häufigsten auftretende Ratio-Wert der normalen Kopienzahl entspricht. Diese Beschränkung ist erforderlich, damit eine Normierung der Ratio-Werte durchgeführt werden kann. Ein solcherart vielfältig aberrantes Eich-Genom, das Monosomien und Trisomien ganzer Chromosomen oder auch von Chromsomenbereichen enthalten könnte, würde zusätzlich die Klassifikation schwer unterscheidbarer Chromosomen erleichtern. Mit einem solchen Eich-Genom könnten auch Abweichungen der Dynamikveränderung von Ratio-Profilen zwischen einzelnen Chromosomenklassen erkannt werden. Dadurch ergeben sich wiederum entsprechend mehr bekannte Ratio-Profile, die einerseits weitere Aussagen über Reproduzierbarkeit und Genauigkeit der durchgeführten Messung erlauben, andererseits eine individuelle Dynamikkorrektur einzelner Chromosomenklassen ermöglichen. Hierbei ist es insbesondere vorteilhaft, daß auch einzelne, häufig abweichende Chromosomenklassen erkannt werden und korrigiert bzw. von der Untersuchung ausgeschlossen werden können.

Ferner können gemäß Anspruch 10 die in Schritt h) aufgrund der bekannten Veränderungen in der Kopienzahl der Eich-Nukleinsäuresequenzen erwarteten Ratio-Werte nicht einem erhaltenen Ratio-Wert, sondern dem Mittelwert aus verschiedenen erhaltenen Ratio-Werten, die von verschiedenen Sequenzen der Eich- und der Referenz-Nukleinsäuresequenzen mit gleichen zu erwartenden Ratio-Werten erhalten worden sind, zugeordnet werden. Durch die Verwendung des Mittelwertes mehrerer Ratio-Werte für die einzelnen Stützstellen zur Ermittlung der Korrekturkurve werden statistische oder systematische chromosomenabhängige Hybridisierungseinflüsse ebenfalls reduziert.

Die Unterdrückung der repetitiven Sequenzen erfolgt nach Anspruch 11 dadurch, daß vor der Hybridisierung die repetitiven Sequenzen der zu untersuchenden Nukleinsäuresequenzen, der Referenz-Nukleinsäuresequenzen und der Eich-Nukleinsäuresequenzen weitgehend entfernt werden. Hierzu sei auf die aus dem Stand der Technik, beispielsweise in der US 5,447,841 bekannten Verfahren hingewiesen.

Alternativ können vor der Hybridisierung die repetitiven Sequenzen der zu untersuchenden Nukleinsäuresequenzen, der Referenz-Nukleinsäuresequenzen und der Eich-Nukleinsäuresequenzen oder die repetitiven Sequenzen der Ziel-Nukleinsäuresequenzen durch Absättigung mit Cot-1-DNA weitgehend blockiert werden. Diese Cot-1-DNA ist unter anderem von Bethesda Research Laboratory (Gaithersburg, MD, USA) erhältlich. Außerdem ist es gemäß der vorliegenden Erfindung möglich, daß die repetitiven Sequenzen der zu untersuchenden Nukleinsäuresequenzen, der Referenz-Nukleinsäuresequenzen und der Eich-Nukleinsäuresequenzen oder die repetitiven Sequenzen der Ziel-Nukleinsäuresequenzen durch Absättigung mit Cot-1-DNA während der Hybridisierung weitgehend blockiert werden.

Diese Verfahren werden beispielsweise in "Optimizing Comparative Genomic Hybridization for Analysis of DNA Sequence Copy Number Changes in Solid Tumors" von Kallionimi et al., Genes, Chromosomes & Cancer, Vol, 10, S. 231 bis 243 (1994) oder in "Image Analysis in Comparative Genomic Hybridization" von Lundsteen et al., Cytometry, Vol. 19, S. 42 bis 50 (1995) beschrieben.

Zu den in Anspruch 1 genannten Verfahrensschritten der Markierung, der Hybridisierung und des Nachweises der Intensitäten der Markierungen, beispielsweise mittels fluoreszenzmikroskopischer Methoden sowie zu den eventuell vorzuschaltenden Verfahrensschritten einer Isolierung und Amplifizierung, sei auf die aus dem Stand der Technik, wie beispielsweise aus der US 5,447,841 oder aus der WO 93/18186, bekannten Methoden verwiesen.

Eine vorteilhafte Ausgestaltung des erfindungsgemäßen Verfahren ist auch die erwähnte Amplifizierung der Nukleinsäuresequenzen des zu untersuchenden Nukleinsäuresequenzkollektivs, des Referenz- und des Eich-Nukleinsäuresequenzkollektivs vor der Markierung, wie in Anspruch 12 angegeben.

Die Korrekturkurven können gemäß Anspruch 13 auch spezifisch für einzelne Chromosomen oder Chromosomenbereiche berechnet werden, falls ein Eich-Nukleinsäuresequenzkollektiv verwendet wird, das für mehrere Chromosomen oder Chromosomenbereiche bekannte Kopienzahlveränderungen aufweist. Die Chromosomen oder Chromosomenbereiche werden dann anhand der spezifischen Korrekturkurven individuell korrigiert. Somit könnte die Dynamikkorrektur auch chromosomenspezifische Dynamikveränderungen ausgleichen.

Eine vorteilhafte Ausgestaltung des erfindungsgemäßen Verfahren ist es auch, wenn die Eich-Nukleinsäuresequenzen Veränderungen in der Kopienzahl in Form von numerischen Chromosomenaberrationen aufweisen.

Weiterhin ist es gemäß Anspruch 15 vorteilhaft, die Eich-Nukleinsäuresequenzen durch Mischen von Bibliotheken ganzer Chromosomen oder Chromosomenbereiche herzustellen.

In Anspruch 16 wird eine zusätzliche vorteilhafte Ausgestaltung für den Fall beschrieben, daß sich die bekannten Veränderungen in der Kopienzahl der Eich-Nukleinsäuresequenzen an geeigneten Stellen befinden. Dann können nämlich die Intensitäten einzelner Chromosomen und/oder Chromosomenbereiche beispielsweise im Karyogramm zur Chromosomenunterscheidung verwendet werden.

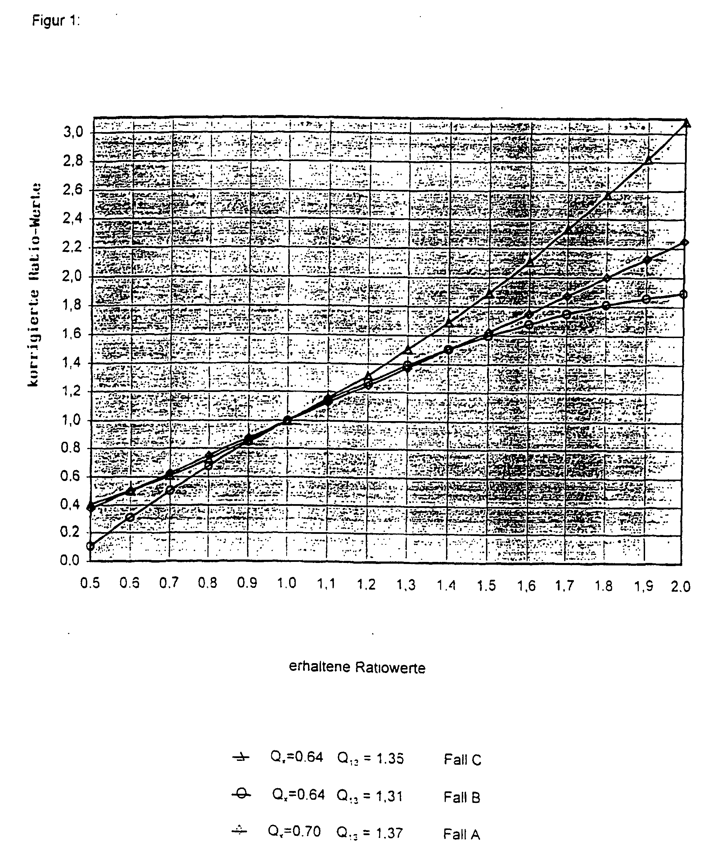

Die Erfindung wird nachstehend ohne Beschränkung des allgemeinen Erfindungsgedankens anhand von Ausführungsbeispielen unter Bezugnahme auf die Figuren 1 bis 3b exemplarisch beschrieben, auf die im übrigen hinsichtlich der Offenbarung aller im Text nicht näher erläuterten erfindungsgemäßen Einzelheiten ausdrücklich verwiesen wird.

Tabelle 1 zeigt beispielhaft die reduzierte Dynamik der Ratio-Werte dreier CGH-Experimente. Das Eich-Nukleinsäuresequenzkollektiv (männlich, Trisomie 13) war mit dem Farbstoff Cy5 markiert, das Referenz-Nukleinsäuresequenzkollektiv (weiblich, normal) mit dem Farbstoff Texas Red. Anstelle der theoretischen Ratio-Werte für X Chromosom und Chromosom 13 von 0.5 und 1.5 wurden Werte zwischen 0.64 und 0.7 bzw. zwischen 1.35 und 1.42 ermittelt.

Tabelle 1:

| Experiment | Mittl. Ratio Chr. X (Anzahl Metaphasen) | Abweichung | Mittl. Ratio Chr. 13 | Abweichung |
|---|---|---|---|---|
| A | 0.70(7) | 0.20 | 1.37 | 0.13 |
| B | 0.64(3) | 0.14 | 1.31 | 0.19 |
| C | 0.64(6) | 0.14 | 1.35 | 0.15 |

Figur 1 zeigt die Korrekturkurven, die sich für die Experimente aus Tabelle 1 bei Anwendung einer quadratischen Korrektur ergeben (vgl III). Aufgrund der Normierung der gemessenen Ratio-Profile schneiden sich die Korrekturkurven immer im Punkt (1.0/1.0).

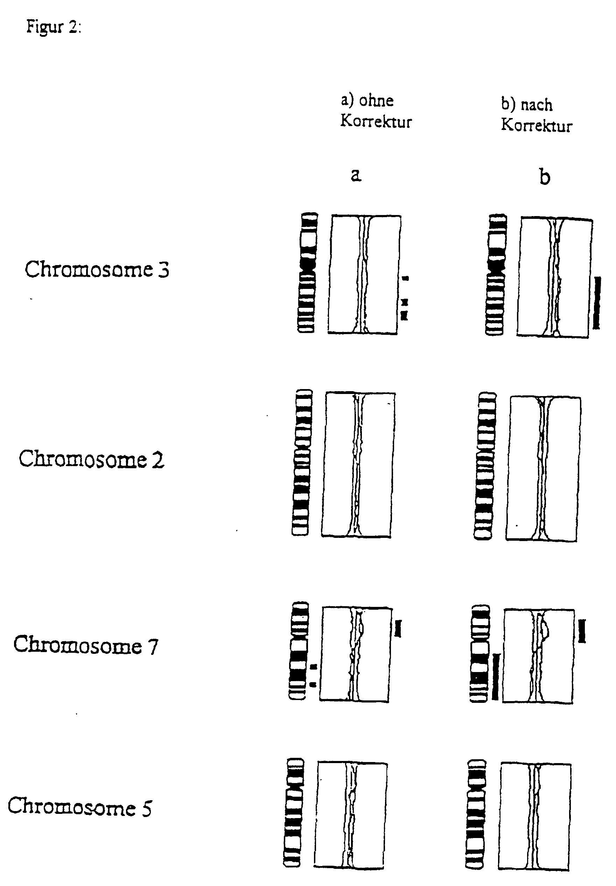

Ein Beispiel für eine Dynamikkorrektur ist in Figur 2 dargestellt. Neben dem Ideogramm ist jeweils das Ratio-Profil von Test-DNA bezogen auf den Mittelwert von Eich- und Referenz-DNA gezeigt. Die Mittellinie entspricht dem Ratio-Wert 1, Amplifikationen der Test-DNA sind als Profilausschläge nach rechts, Deletionen als Profilausschläge nach links zu erkennen. Die Signifikanzgrenzen sind als zur Mittellinie symmetrische Kurven dargestellt. Profilbereiche, die rechts bzw. links der Signifikanzgrenzen liegen, werden als Amplifikationen bzw. Deletionen des Test-Genoms interpretiert. Diese überschwelligen Profilbereiche sind durch schwarze Balken zusätzlich hervorgehoben. Chromosom 3 weist eine Amplifikation 3q auf, die ohne Korrektur (a) an der Grenze des Signifikanzintervalls verläuft. Nach erfolgter Dynamikkorrektur (b) ist die Amplifikation 3q signifikant. Das normale Chromosom 2 (unten) bleibt auch nach der Korrektur innerhalb der Signifikanzgrenzen.

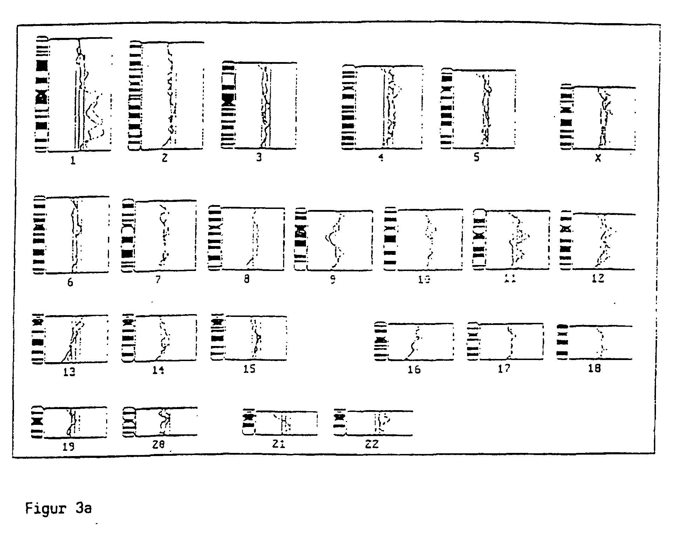

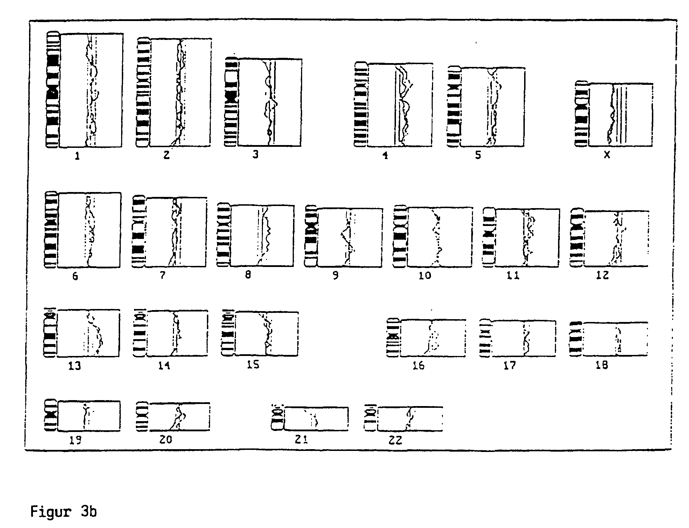

Die einfachste Möglichkeit, die zusätzliche Information des Eich-Nukleinsäuresequenzkollektivs zu nutzen, ist die Darstellung der Ratioprofile von Test-Nukleinsäuresequenzkollektiv gegen Referenz- und Eich-Nukleinsäuresequenzkollektiv einerseits (Fig. 3a) und der Ratioprofile von Eich-Nukleinsäuresequenzkollektiv gegen Referenz-Nukleinsäuresequenzkollektiv (Fig. 3b) für einzelne Metaphasen. Die Kopienzahlveränderung des Test-Nukleinsäuresequenzkollektivs für Chromosom 1q ist klar erkennbar. Ebenfalls erkennbar sind Kopienzahländerungen (z.B. Chromosom 19), die aufgrund geringer Qualität der Hybridisierung im Bereich der Signifikanzschwelle liegen. Die Ratioprofile Eich- gegen Referenz-Nukleinsäuresequenzkollektiv (Fig. 3b) sollten theoretisch bei 1.0 bzw. 0.5 (X-Chromosom) und 1.5 (Chromosom 13) liegen. Chromosom 19 zeigt einen Hybridisierungsartefakt und sollte für die Auswertung nicht berücksichtigt werden. Die erkennbaren Abweichungen (Ratioprofil X Chromosom 0.6 bis 0.7, Chromosom 13 maximal 1.5) spiegeln die Dynamikverringerung wider.

Ein weiterer Gegenstand der vorliegenden Erfindung ist ein diagnostischer Kit zum Nachweis von Änderungen in vorstehend definierten Nukleinsequenzkollektiven (T) als Zielmoleküle bzw. Test-DNA, enthaltend mindestens ein Referenz-Nukleinsequenzkollektiv (R) und ein Eich-Nukleinsäuresequenzkollektiv (S) gemäß vorstehender Definitionen.

Der erfindungsgemäße Kit kann insbesondere zum Nachweis bzw. Ausschluß von chromosomalen Abänderungen in der Humangenetik, wie balancierter Chromosomenrearrangements, die bekanntermaßen von großer Bedeutung für die Verwirklichung von Kinderwunsch bei Trägern einer solchen Veränderung sind, balancierter und unbalancierter Chromosenveränderungen als Ursache für Fehlbildungen und/oder mentaler Retardierung, und in der Tumordiagnostik sowohl solider Tumore als auch hämatologischer Neoplasien (AML, ALL, MDS u.a.) einerseits zur Erfassung bekannter, prognoserelevanter Veränderungen andererseits zur Bestimmung weiterer, bisher unbekannter Veränderungen eingesetzt werden.

**Patentansprüche**

1. Verfahren zum Vergleich der Kopienzahl von Nukleinsäuresequenzen zweier oder mehrerer Nukleinsäuresequenzkollektive mit folgenden Schritten:

   a) Hybridisierung von

       i) einem zu untersuchenden Nukleinsäuresequenzkollektiv (T),
       ii) einem Referenz-Nukleinsäuresequenzkollektiv (R) und
       iii) mindestens einem weiteren Nukleinsäuresequenzkollektiv mit bekannter Abweichung in der Anzahl der Kopien der Nukleinsäuresequenzen an mindestens einer bekannten Stelle (Eich-Nukleinsäuresequenzkollektiv, S),
       wobei das weitere Nukleinsäuresequenzkollektiv dem Referenz-Nukleinsäuresequenzkollektiv möglichst ähnlich ist,

   wobei die Nukleinsäuresequenzkollektive mit unterscheidbaren Markierungen versehen sind, auf ein Kollektiv geeigneter Ziel-Nukleinsäuresequenzen,
   b) Nachweis der Intensitäten der Markierungen der einzelnen, auf die Ziel-Nukleinsäuresequenzen hybridisierten markierten Nukleinsäuresequenzkollektive,
   c) Bestimmung der Quotienten $Q_R$ der Intensitäten der Markierungen der Nukleinsäuresequenzen des zu untersuchenden Nukleinsäuresequenzkollektivs und den entsprechenden Intensitäten der Markierungen der Nukleinsäuresequenzen des Referenz-Nukleinsäuresequenzkollektivs,
   d) Bestimmung der Quotienten $Q_S$ der Intensitäten der Markierungen der Nukleinsäuresequenzen des zu untersuchenden Nukleinsäuresequenzkollektivs und den entsprechenden Intensitäten der Markierungen der Nukleinsäuresequenzen des Eich-Nukleinsäuresequenzkollektivs,
   e) Bestimmung der relativen Kopienzahlen, der sogenannten Ratio-Werte, von den zu untersuchenden Nukleinsäuresequenzen gegenüber den Referenz-Nukleinsäuresequenzen durch geeignete Normierung der Quotienten $Q_R$ aus c),
   f) Bestimmung der relativen Kopienzahlen, der sogenannten Ratio-Werte, von den zu untersuchenden Nukleinsäuresequenzen gegenüber den Eich-Nukleinsäuresequenzen durch geeignete Normierung der Quotienten $Q_S$ aus d),
   g) Bestimmung von wenigstens zwei Stützstellen, die wenigstens zwei im Betrag voneinander abweichenden Ratio-Werten der Eich- und der Referenz-Nukleinsäuresequenzen mit bekannter Abweichung in der Kopienzahl der Eich-Nukleinsäuresequenzen entsprechen,
   h) Berechnung einer Korrekturkurve anhand der an den Stützstellen vorhandenen Abweichungen der erhaltenen Ratio-Werte von den aufgrund der bekannten Veränderungen in der Kopienzahl der Eich-Nukleinsäuresequenzen erwarteten Ratio-Werten, und

i) Korrektur der unter e) erhaltenen Kopienzahlen bzw. Ratio-Werte mit Hilfe der unter h) erhaltenen Korrekturkurve.

2. Verfahren nach Anspruch 1,
**dadurch gekennzeichnet, daß** die Markierung der Nukleinsäuresequenzen mit unterschiedlichen Fluoreszenzfarbstoffen und daß der Nachweis der Intensitäten der Markierungen durch Messung der fluoreszenzmikroskopischen Signale der markierten Nukleinsäuresequenzen erfolgt.

3. Verfahren nach einem der Ansprüche 1 oder 2,
**dadurch gekennzeichnet, daß** als Kollektiv der Ziel-Nukleinsäuresequenzen Metaphase-Chromosomen verwendet werden.

4. Verfahren nach einem der Ansprüche 1 oder 2,
**dadurch gekennzeichnet, daß** als Kollektiv der Ziel-Nukleinsäuresequenzen isolierte, auf einem Substrat in definierter Weise angeordnete Nukleinsäuresequenzen verwendet werden.

5. Verfahren nach einem der Ansprüche 1 bis 4,
**dadurch gekennzeichnet, daß** als Kollektiv der Ziel-Nukleinsäuresequenzen DNA verwendet wird.

6. Verfahren nach einem der Ansprüche 1 bis 4,
**dadurch gekennzeichnet, daß** als Kollektiv der Ziel-Nukleinsäuresequenzen cDNA verwendet wird.

7. Verfahren nach einem der Ansprüche 1 bis 6,
**dadurch gekennzeichnet, daß** als zu untersuchendes Nukleinsäuresequenzkollektiv, als Referenz-Nukleinsäuresequenzkollektiv und als Eich-Nukleinsäuresequenzkollektiv genomische DNA verwendet wird.

8. Verfahren nach einem der Ansprüche 1 bis 7,
**dadurch gekennzeichnet, daß** als zu untersuchende Nukleinsäuresequenzen mRNA oder cDNA aus Testzellen, und daß als Referenz- und als Eich-Nukleinsäuresequenzen mRNA oder cDNA aus Referenz- bzw. Eich-Zellen verwendet wird.

9. Verfahren nach einem der Ansprüche 1 bis 8,
**dadurch gekennzeichnet, daß** für den Schritt g) als Stützstellen mehr als zwei im Betrag voneinander abweichende Ratio-Werte verwendet werden, und somit Korrekturkurven höherer Ordnung berechnet werden können.

10. Verfahren nach einem der Ansprüche 1 bis 9,
**dadurch gekennzeichnet, daß** den in Schritt h) aufgrund der bekannten Veränderungen in der Kopienzahl der Eich-Nukleinsäuresequenzen erwarteten Ratio-Werten nicht ein erhaltener Ratio-Wert, sondern der Mittelwert aus verschiedenen erhaltenen Ratio-Werten, die von verschiedenen Sequenzen der Eich- und der Referenz-Nukleinsäuresequenzen mit gleichen zu erwartenden Ratio-Werten erhalten worden sind, zugeordnet wird.

11. Verfahren nach einem der Ansprüche 1 bis 10,
**dadurch gekennzeichnet, daß** eine Unterdrückung der repetitiven Sequenzen dadurch erfolgt, daß vor der Hybridisierung die repetitiven Sequenzen der zu untersuchenden Nukleinsäuresequenzen, der Referenz-Nukleinsäuresequenzen und der Eich-Nukleinsäuresequenzen weitgehend entfernt werden, oder
daß vor der Hybridisierung die repetitiven Sequenzen der zu untersuchenden Nukleinsäuresequenzen, der Referenz-Nukleinsäuresequenzen und der Eich-Nukleinsäuresequenzen oder die repetitiven Sequenzen der Ziel-Nukleinsäuresequenzen durch Absättigung mit Cot-1-DNA weitgehend blockiert werden, oder
daß die repetitiven Sequenzen der zu untersuchenden Nukleinsäuresequenzen, der Referenz-Nukleinsäuresequenzen und der Eich-Nukleinsäuresequenzen oder die repetitiven Sequenzen der Ziel-Nukleinsäuresequenzen durch Absättigung mit Cot-1-DNA während der Hybridisierung weitgehend blockiert werden.

12. Verfahren nach einem der Ansprüche 1 bis 11,
**dadurch gekennzeichnet, daß** die zu untersuchenden, die Referenz- und die Eich-Nukleinsäuresequenzen vor der Markierung amplifiziert werden.

13. Verfahren nach einem der Ansprüche 1 bis 12,
    **dadurch gekennzeichnet, daß** die Eich-Nukleinsäuresequenzen so gewählt sind, daß spezifische Korrekturkurven für einzelne Chromosomen oder Chromosomenbereiche berechnet werden können und die Chromosomen oder Chromosomenbereiche anhand der spezifischen Korrekturkurven individuell korrigiert werden.

14. Verfahren nach einem der Anprüche 1 bis 13,
    **dadurch gekennzeichnet, daß** die Eich-Nukleinsäuresequenzen aus einem Genom mit Veränderungen in der Kopienzahl in Form von numerischen Chromosomenaberrationen stammen.

15. Verfahren nach einem der Ansprüche 1 bis 14,
    **dadurch gekennzeichnet, daß** die Eich-Nukleinsäuresequenzen durch Mischen von Bibliotheken ganzer Chromosomen oder Chromosomenbereiche hergestellt werden.

16. Verfahren nach einem der Ansprüche 1 bis 15,
    **dadurch gekennzeichnet, daß** die bekannten Veränderungen in der Kopienzahl der Eich-Nukleinsäuresequenzen sich an geeigneten Stellen befinden, so daß die Intensitätsunterschiede der Markierung der Eich-Nukleinsäuresequenzen bei Verwendung von Metaphase-Chromosomen als Ziel-Nukleinsäuresequenzen zusätzlich zur Unterscheidung von Chromosomen verwendet werden können.

17. Diagnostischer Kit zum Nachweis von Änderungen in einem zu untersuchenden Nukleinsäuresequenzkollektiv (T) gemäß dem Verfahren in Ansprüchen 1-16, enthaltend mindestens ein Referenz-Nukleinsäuresequenzkollektiv (R) und ein Eich-Nukleinsäuresequenzkollektiv (S) gemäß Definition in Anspruch 1.

18. Verwendung des Kits nach Anspruch 17 zum Nachweis von chromosomalen Abänderungen in der Humangenetik und Tumordiagnostik.


**Claims**

1. Method for comparing the copy number of nucleic acid sequences belonging to two or more nucleic acid sequence groups, comprising the following steps:

   a) hybridizing

       i) a nucleic acid sequence group which is to be investigated (T),
       ii) a reference nucleic acid sequence group (R), and
       iii) at least one additional nucleic acid sequence group having a known difference in the number of copies of the nucleic acid sequences at at least one known site (calibration nucleic acid sequence group, S), with the additional nucleic acid sequence group being as similar as possible to the reference nucleic acid sequence group,

   with the nucleic acid sequence groups being provided with distinguishable labels, to a group of suitable target nucleic acid sequences,
   b) detecting the intensities of the labels of the individual labelled nucleic acid sequence groups which are hybridized to the target nucleic acid sequences,
   c) determining the quotients $Q_R$ of the intensities of the labels of the nucleic acid sequences of the nucleic acid sequence group to be investigated and the corresponding intensities of the labels of the nucleic acid sequences of the reference nucleic acid sequence group,
   d) determining the quotients $Q_S$ of the intensities of the labels of the nucleic acid sequences of the nucleic acid sequence group to be investigated and the corresponding intensities of the labels of the nucleic acid sequences of the calibration nucleic acid sequence group,
   e) using suitable standardization of the quotients $Q_R$ from c) to determine the relative copy numbers, i.e. what are termed the ratio values, of the nucleic acid sequences to be investigated vis-à-vis the reference nucleic acid sequences,
   f) using suitable standardization of the quotients $Q_S$ from d) to determine the relative copy numbers, i.e. what are termed the ratio values, of the nucleic acid sequences to be investigated vis-à-vis the calibration nucleic acid sequences,
   g) determining at least two datum points which correspond to at least two ratio values, which differ from each other in amount, of the calibration and reference nucleic acid sequences, with known difference in the copy number of the calibration nucleic acid sequences,

h) calculating a correction curve, using the differences, which are present at the datum points, in the ratio values obtained, of the ratio values which are expected on a basis of the known differences in the copy number of the calibration nucleic acid sequences, and

i) using the correction curve obtained under h) to correct the copy numbers or ratio values obtained under e).

2. Method according to Claim 1,
   **characterized in that** the nucleic acid sequences are labelled with different fluorescence dyes and **in that** the intensities of the labels are detected by measuring the fluorescence-microscopic signals of the labelled nucleic acid sequences.

3. Method according to either Claim 1 or 2,
   **characterized in that** metaphase chromosomes are used as the group of target nucleic acid sequences.

4. Method according to either Claim 1 or 2,
   **characterized in that** isolated nucleic acid sequences, which are arranged in a defined manner on a substrate, are used as the group of target nucleic acid sequences.

5. Method according to any one of Claims 1 to 4,
   **characterized in that** DNA is used as the group of target nucleic acid sequences.

6. Method according to any one of Claims 1 to 4,
   **characterized in that** cDNA is used as the group of target nucleic acid sequences.

7. Method according to any one of Claims 1 to 6,
   **characterized in that** genomic DNA is used as the nucleic acid sequence group to be investigated, as the reference nucleic acid sequence group and as the calibration nucleic acid sequence group.

8. Method according to any one of Claims 1 to 7,
   **characterized in that** mRNA or cDNA from test cells is used as nucleic acid sequences to be investigated and **in that** mRNA or cDNA from reference cells and calibration cells is used as reference nucleic acid sequences and as calibration nucleic acid sequences, respectively.

9. Method according to any one of Claims 1 to 8,
   **characterized in that** more than two ratio values differing from each other in amount are used as datum points for step g) and that correction curves of a higher order can consequently be calculated.

10. Method according to any one of Claims 1 to 9,
    **characterized in that** the mean value of different ratio values which have been obtained, which ratio values have been obtained from different sequences of the calibration and the reference nucleic acid sequences having ratio values expected to be identical, rather than a ratio value which has been obtained, is assigned to the ratio values which are expected in step h) on the basis of the known changes in the copy number of the calibration nucleic acid sequences.

11. Method according to any one of Claims 1 to 10,
    **characterized in that** the repetitive sequences are eliminated by extensively removing the repetitive sequences of the nucleic acid sequences to be investigated, of the reference nucleic acid sequences and of the calibration nucleic acid sequences prior to the hybridization, or by extensively blocking the repetitive sequences of the nucleic acid sequences to be investigated, of the reference nucleic acid sequences and of the calibration nucleic acid sequences, or the repetitive sequences of the target nucleic acid sequences, prior to the hybridization by saturating with Cot-1 DNA, or by extensively blocking the repetitive sequences of the nucleic acid sequences to be investigated, of the reference nucleic acid sequences and of the calibration nucleic acid sequences, or the repetitive sequences of the target nucleic acid sequences, by saturating with Cot-1 DNA during the hybridization.

12. Method according to any one of Claims 1 to 11,
    **characterized in that** the nucleic acid sequences to be investigated, the reference nucleic acid sequences and the calibration nucleic acid sequences are amplified prior to labelling.

13. Method according to any one of Claims 1 to 12,
**characterized in that** the calibration nucleic acid sequences are selected such that specific correction curves can be calculated for individual chromosomes or chromosome regions and the chromosomes or chromosome regions are corrected individually using the specific correction curves.

14. Method according to any one of Claims 1 to 13,
**characterized in that** the calibration nucleic acid sequences are derived from a genome having changes in the copy number in the form of numeric chromosome aberrations.

15. Method according to any one of Claims 1 to 14,
**characterized in that** the calibration nucleic acid sequences are prepared by mixing libraries of whole chromosomes for chromosome regions.

16. Method according to any one of Claims 1 to 15,
**characterized in that** the known changes in the copy number of the calibration nucleic acid sequences are located at suitable sites such that the differences in the intensity of the labelling of the calibration nucleic acid sequences when using metaphase chromosomes as target nucleic acid sequences can additionally be used for distinguishing chromosomes.

17. Diagnostic kit for detecting changes in a nucleic acid sequence group to be investigated (T) according to the method in Claims 1-16, which comprises at least one reference nucleic acid sequence group (R) and one calibration nucleic acid sequence group (S) in accordance with the definition in Claim 1.

18. Use of the kit according to Claim 17 for detecting chromosomal alterations in human genetics and tumour diagnosis.


**Revendications**

1. Procédé pour comparer le nombre de copies de séquences d'acides nucléiques de deux ou plusieurs collectifs de séquences d'acides nucléiques, comprenant les étapes suivantes :

   a) hybridation sur un collectif de séquences cibles d'acide nucléique appropriées

      i) d'un collectif (T) de séquences d'acide nucléique à examiner,
      ii) d'un collectif (R) de séquences d'acide nucléique de référence et
      iii) d'au moins un autre collectif de séquences d'acide nucléique, aussi semblable que possible au collectif de référence de séquences d'acide nucléique, présentant un écart connu dans le nombre de copies des séquences d'acides nucléiques en au moins un site connu (collectif S de séquences d'acide nucléique étalon),

   les collectifs de séquences d'acides nucléiques étant pourvus de marquages différenciables,
   b) détection des intensités des marquages des collectifs individuels de séquences d'acides nucléiques marqués et hybridés sur les séquences cibles d'acides nucléiques,
   c) détermination des quotients $Q_R$ des intensités des marquages des séquences d'acide nucléique du collectif de séquences d'acide nucléique à examiner et des intensités correspondantes des marquages des séquences d'acide nucléique du collectif de séquences d'acide nucléique de référence,
   d) détermination des quotients $Q_S$ des intensités des marquages des séquences d'acide nucléique du collectif de séquences d'acide nucléique à examiner et des intensités correspondantes des marquages des séquences d'acide nucléique du collectif de séquences d'acide nucléique étalon,
   e) détermination des nombres relatifs de copies, autrement dit des valeurs de ratio, des séquences d'acides nucléiques à examiner par rapport aux séquences d'acides nucléiques de référence par normalisation appropriée des quotients $Q_R$ de c),
   f) détermination des nombres relatifs de copies, c'est-à-dire des valeurs de ratio, des séquences d'acides nucléiques à examiner par rapport aux séquences d'acides nucléiques étalons par normalisation appropriée des quotients $Q_S$ de d),
   g) détermination d'au moins deux sites de base, qui correspondent à au moins deux ratios, dont les valeurs s'écartent l'une de l'autre, des séquences d'acides nucléiques étalons et de référence avec un écart connu dans le nombre de copies des séquences d'acides nucléiques étalons,

h) calcul de la courbe de correction à l'aide des écarts existant au niveau des sites de base des valeurs de ratio obtenues par rapport aux valeurs de ratio attendues sur la base des variations connues du nombre de copies des séquences d'acides nucléiques étalons, et

i) correction du nombre de copies obtenu en e), autrement dit des valeurs de ratio à l'aide de la courbe de correction obtenue en h).

2. Procédé suivant la revendication 1, **caractérisé en ce que** le marquage des séquences d'acides nucléiques est effectué avec des colorants fluorescents différents et **en ce que** la détection des intensités des marquages est effectuée par mesure des signaux de microscopie en fluorescence des séquences d'acides nucléiques marquées.

3. Procédé suivant les revendications 1 et 2, **caractérisé en ce qu'**on utilise des chromosomes en métaphase comme collectif des séquences cibles d'acide nucléique.

4. Procédé suivant l'une des revendications 1 et 2, **caractérisé en ce qu'**on utilise comme collectif des séquences cibles d'acide nucléique, des séquences d'acides nucléiques isolées, disposées de manière définie sur un substrat.

5. Procédé suivant l'une des revendications 1 à 4, **caractérisé en ce qu'**on utilise de l'ADN comme collectif des séquences cibles d'acide nucléique.

6. Procédé suivant l'une des revendications 1 à 4, **caractérisé en ce qu'**on utilise de l'ADNc comme collectif des séquences cibles d'acide nucléique.

7. Procédé suivant l'une des revendications 1 à 6, **caractérisé en ce qu'**on utilise de l'ADN génomique comme collectif des séquences d'acide nucléique à examiner, comme collectif des séquences d'acide nucléique de référence et comme collectif des séquences d'acide nucléique étalon.

8. Procédé suivant l'une des revendications 1 à 7, **caractérisé en ce qu'**on utilise de l'ARNm ou de l'ADNc provenant de cellules testées comme séquences d'acides nucléiques à examiner et **en ce qu'**on utilise comme séquences d'acides nucléiques de référence et comme séquences d'acides nucléiques étalons de l'ARNm ou de l'ADNc provenant respectivement de cellules de référence et de cellules étalons.

9. Procédé suivant l'une des revendications 1 à 8, **caractérisé en ce qu'**on peut utiliser comme sites de base pour l'étape g) plus de deux ratios ayant des valeurs s'écartant l'une de l'autre et par conséquent on peut calculer des courbes de correction d'un ordre plus haut.

10. Procédé suivant l'une des revendications 1 à 9, **caractérisé en ce qu'**on attribue aux valeurs de ratio attendues dans l'étape h) sur la base des variations connues du nombre de copies des séquences d'acides nucléiques étalons non pas une valeur de ratio obtenue, mais la valeur moyenne de diverses valeurs de ratio qui ont été obtenues de diverses séquences étalons et séquences de référence d'acides nucléiques ayant des valeurs de ratio escomptées égales.

11. Procédé suivant l'une des revendications 1 à 10, **caractérisé en ce qu'**on effectue une suppression des séquences répétitives en éliminant largement avant l'hybridation les séquences répétitives des séquences d'acides nucléiques à examiner, des séquences d'acides nucléiques de référence et des séquences d'acides nucléiques étalons, ou

**en ce qu'**on bloque largement avant l'hybridation les séquences répétitives des séquences d'acides nucléiques à examiner, des séquences d'acides nucléiques de référence et des séquences d'acides nucléiques étalons ou les séquences répétitives des séquences d'acides nucléiques cibles par saturation avec de l'ADN de Cot-1, ou **en ce qu'**on bloque largement les séquences répétitives des séquences d'acides nucléiques à examiner, des séquences d'acides nucléiques de référence et des séquences d'acides nucléiques étalons ou les séquences répétitives des séquences d'acides nucléiques cibles par saturation avec de l'ADN de Cot-1 pendant l'hybridation.

12. Procédé suivant l'une des revendications 1 à 11, **caractérisé en ce que** les séquences d'acides nucléiques à examiner, de référence et étalons sont amplifiées avant le marquage.

13. Procédé suivant l'une des revendications 1 à 12, **caractérisé en ce qu'**on choisit les séquences d'acides nucléiques étalons de manière à pouvoir calculer des courbes spécifiques de correction pour les chromosomes individuels ou les domaines individuels de chromosomes et on corrige individuellement les chromosomes ou les domaines de chromosomes à l'aide des courbes spécifiques de correction.

**14.** Procédé suivant l'une des revendications 1 à 13, **caractérisé en ce que** les séquences d'acides nucléiques étalons proviennent d'un génome présentant des variations du nombre de copies sous forme d'aberrations numériques des chromosomes.

**15.** Procédé suivant l'une des revendications 1 à 14, **caractérisé en ce que** les séquences d'acides nucléiques étalons sont établies par mélange de bibliothèques de chromosomes entiers ou de régions de chromosomes.

**16.** Procédé suivant l'une des revendications 1 à 15, **caractérisé en ce que** les modifications connues du nombre de copies des séquences d'acides nucléiques étalons se trouvent en des sites appropriés, si bien que les différences d'intensités du marquage des séquences d'acides nucléiques étalons peuvent être exploitées en outre pour différencier des chromosomes lors de l'utilisation de chromosomes en métaphase comme séquences d'acides nucléiques cibles.

**17.** Kit diagnostique pour la détection de variations dans un collectif (T) de séquences d'acide nucléique à examiner conformément au procédé selon les revendications 1 à 16, contenant au moins un collectif (R) de séquences d'acide nucléique de référence et un collectif (S) de séquences d'acide nucléique étalon selon la définition donnée dans la revendication 1.

**18.** Utilisation du kit suivant la revendication 17 pour la détection de modifications chromosomiques en génétique humaine et dans le diagnostic des tumeurs.

Figur 1:

erhaltene Ratiowerte

| | | | |
|---|---|---|---|
| ⊥ | $Q_r$=0.64 | $Q_{13}$ = 1.35 | Fall C |
| ⊖ | $Q_x$=0.64 | $Q_{13}$ = 1.31 | Fall B |
| ÷ | $Q_x$=0.70 | $Q_{13}$ = 1.37 | Fall A |

Figur 2:

Figur 3a

Figur 3b